Europäisches Patentamt

European Patent Office

Office européen des brevets

(19)

(11) Publication number: **0 132 292**
A2

(12) **EUROPEAN PATENT APPLICATION**

(21) Application number: 84304093.2

(22) Date of filing: 18.06.84

(51) Int. Cl.⁴: **G 01 N 33/542,** G 01 N 33/577,
G 01 N 33/68, G 01 N 33/74,
G 01 N 33/78, G 01 N 33/88,
G 01 N 33/92, G 01 N 33/576,
G 01 N 33/574

(30) Priority: 27.06.83 JP 114357/83

(43) Date of publication of application: 30.01.85
Bulletin 85/5

(84) Designated Contracting States: CH DE FR GB IT LI NL

(71) Applicant: FUJIREBIO KABUSHIKI KAISHA also trading
as FUJIREBIO INC., 6-7, Shimoochiai 4-chome
Shinjuku-ku, Tokyo 161 (JP)

(72) Inventor: Ashihara, Yoshihiro, 2-402, Fuchudanchi 28-1,
Harumicho 1-chome, Fuchu-shi Tokyo (JP)
Inventor: Kasahara, Yasushi, 310, 4-4,
Nagayama 3-chome, Tama-shi Tokyo (JP)

(74) Representative: Silverman, Warren et al, HASELTINE
LAKE & CO. Hazlitt House 28 Southampton Buildings
Chancery Lane, London WC2A 1AT (GB)

(54) Method of measuring biological ligands.

(57) A highly sensitive and specific method of measuring a
biological ligand which is suitable as a clinical test for
determining physiological substances and trace com-
ponents in humoral fluid comprises allowing there to coexist
in an aqueous medium a system comprising (A) a ligand (1)
to be measured, (B) a combination of a biotinyl enzyme
inhibitor and a ligand (2) having an antigenic determinant
which is also an antigenic determinant of ligand (1) and (C)
an antibody capable of reacting with said antigenic determinant, contacting said system with a biotinyl enzyme, measuring the residual biotinyl enzyme activity and utilising the
measurement thereby obtained as a measure of the biologi-
cal ligand.

EP 0 132 292 A2

"METHOD OF MEASURING BIOLOGICAL LIGANDS"

This invention relates to a method of measuring a biological ligand, for example, a medicinal substance or a trace compound resulting from one of various diseases and to be found in a humoral fluid such as blood serum or urine.

The measurement of the concentration in a body (or humoral) fluid, in particular the blood, of a drug such as digoxin or theophylline, administered to a patient, is important for the appropriate treatment. The detection in the blood of a patient of trace components derived from various diseases such as cancer is important in early diagnoses of the diseases.

Accordingly, various methods have been developed for detecting these trace components in blood. Among these, enzyme immunoassay has come to be widely employed because of its high sensitivity, specificity and capability for rapid processing of a large number of samples. However, the sensitivity is not sufficient with conventional enzyme immunoassay, and it is not easy to determine concentrations exactly because of variations in results which may arise owing to washing procedures and transferring of tubes.

It is an object of this invention to provide a simple method of measuring a biological ligand which allows exact concentrations to be measured with high sensitivity of trace components in blood.

According to the present invention, there is provided a method of measuring a biological ligand, characterised by allowing there to coexist in an aqueous medium a system comprising (A) a ligand (1) to be measured, (B) a combination of a biotinyl enzyme inhibitor and a ligand (2) having an antigenic determinant which is also an antigenic determinant of ligand (1) and (C) an antibody capable of reacting with said antigenic determinant, contacting said system with a biotinyl enzyme, measuring the residual biotinyl enzyme activity and utilizing the measurement thereby obtained as a measure of the biological ligand.

This method is one of several devised by the present inventors for measuring biological ligands. In one such method described in European Patent application No.84301154.5, the method for measuring a biological ligand comprises bringing into contact with each other in aqueous solution, (a) the ligand to be measured, (b) an enzyme or a combination of an enzyme and a macromolecular substance, and (c) either a combination of an antibody against said ligand and an antibody against said enzyme, or a combination of an antibody against said ligand, an antibody against said enzyme and a macromolecular substance, determining the residual activity of said enzyme and utilising the result obtained as a measure of the ligand.

Another such method which is described in our European Patent Application No.84302853.1 is characterised by allowing there to coexist in an aqueous medium:

(A)    a biologically active composition comprising firstly an immobilised antibody phase whose antibody is capable of reacting with a ligand (1) to be measured or an immobilised phase of a biological ligand (2) which is capable of reacting with said antibody and secondly an immobilised biotinyl enzyme phase or immobilised biotinyl enzyme inhibitor phase,

(B)    a water-soluble material combination of whichever of said ligand (2) or said antibody on the one hand and a biotinyl enzyme inhibitor or a biotinyl enzyme on the other hand are absent from the biologically active composition, the biotinyl enzyme inhibitor then being one which is capable of reacting with the biotinyl enzyme of the biologically active composition or the biotinyl enzyme then being one which is capable of reacting with the biotinyl enzyme inhibitor of the biologically active composition, and

(C) the ligand (1) to be measured,

and measuring the residual biotinyl enzyme activity or the residual biotinyl enzyme inhibitory activity of said biologically active composition or said aqueous solution and utilising the result as a measure of the ligand.

The subject to be measured by the method of this invention is a ligand (1). The ligand (1) is a substance having one or more antigenic determinants, and may be a synthetic drug, for example digoxin, theophylline or phenytoin, an antibiotic, for example penicillin, amikacin or gentamicin, or may be insulin, TSH, T4, prostaglandin, IgG, $\alpha$-fetoprotein, glycolipids, HBs antigen, or a carcinoembryonic antigen. There is no restriction on the nature of the fluid containing the ligand (1) and it may be serum or urine.

Ligand (2) also has one or more antigenic determinants, and at least one antigenic determinant is an antigenic determinant of ligand (1). Indeed the antigenic determinants of ligand (2) may be identical with those of ligand (1), and accordingly, ligand (2) may be identical with ligand (1).

The biotinyl enzyme inhibitor is, for example, avidin, streptoavidin or a derivative thereof capable of binding to biotin (hereinafter together referred to as avidin etc). Such derivatives include avidin or streptoavidin modified with general chemical modifiers such as acetylating agents. The coupling constants of the derivatives are preferably small.

The method of binding ligand (2) to the biotinyl enzyme inhibitor may be selected in accordance with the functional groups of both substances. Such functional groups include, amino groups, carboxyl groups, hydroxyl groups, thiol groups, imidazole groups and phenyl groups.

For example, when ligand (2) is a low molecular weight molecule and has a carboxyl group, it is possible to use a method where the carboxyl group is converted to a succinimido ester. This ester group is an active group, and reacts with an amino group of avidin etc. to form a stable peptide bond.

When ligand (2) has a thiol group, a thiol group is introduced into avidin etc. using S-acetylmercaptosuccinic anhydride, and both thiol groups are bound by using a thiol-reacting bifunctional cross-linking reagent to produce the required combination.

The amino group of avidin etc. may be bound to an amino group of ligand (2) by many methods including the diisocyanate method, the glutaraldehyde method, the difluorobenzene method and the benzoquinone method. When binding an amino group and a carboxyl group, it is possible to use the carbodiimide method or the Woodward reagent method. The periodate oxidation method (Nakane method) where a bridge between an amino group and a sugar chain is formed can also be utilised where appropriate. When a phenyl group is present, the diazotization and the alkylation coupling methods are available. The ligand (2) avidin etc. combination employed in the method of the invention may be synthesized by any of these methods as appropriate.

After the reaction, the combination produced is purified by gel filtration, ion-exchange chromatography, adsorption chromatography or thin-layer chromatography using silica gel or a porous polymer, and lyophilized, if necessary.

The antibody against the antigenic determinant common to ligand (1) and to ligand (2) may include a fragment of an immunoglobulin, for example $F(ab')_2$, Fab' or Fab.

The antibody may be produced by following any convenient method for producing an antibody. For example, ligand (1), ligand (2), the aforesaid ligand (2) - avidin etc. combination or a conjugate of either of these ligands and a protein material is injected once or several times into the subcutaneous region of the back, foot pad or femoral muscle of a warm-blooded animal such as a rabbit, goat, horse, guinea pig or chicken, in an amount of from 0.3 to 2 mg per kg together with an adjuvant, thereby producing the antibody in the humoral fluid such as serum. This humoral fluid may be used as such as a supply source for the antibody. However, the antibody is preferably separated by following a conventional isolation method for an immunoglobulin.

Alternatively, the antibody may be produced as a monoclonal antibody. In this case, an antigen for the antibody is injected several times into the abdominal cavity of a mouse together with an adjuvant, and its spleen is excised. The spleen cell is fused with a mouse myeloma cell by a conventional method involving the use of polyethylene glycol. The hybridoma thus obtained is cultured and cloned, and a cell capable of producing the desired antibody is obtained. This cell is injected into the abdominal cavity of a mouse, and multiplied. Then, ascites are collected, and the desired antibody is separated from the ascites.

When carrying out the method of this invention, ligand (1) contained in a sample and the above combination are allowed to make contact with this antibody in a solution. The amount of the aforesaid combination used is preferably equal to the maximum amount of the ligand (1) to be expected. The amount of the antibody employed will vary according to its coupling constant with respect to the combination etc. and usually it is preferred to use 1 to 5 moles of antibody per mole of the combination. The amount of ligand (1) which is present in a test sample is suitably from 0.01 to 1 mg, and in this case, the combination used is suitably from 1 ng to 1 mg, and that of the antibody is from 1 μg to 10

mg. The pH of the solution is preferably kept in the range of from 4 to 8.5, and a buffer solution such as a phosphate buffer solution or an acetate buffer solution may be employed. The working temperature used also may vary according to the identity of each of the ligand (1), the combination and the antibody, and it is usually in the range of from 20 to $45^{o}$C. The contacting times of the ligand (1) with the antibody and the combination with the antibody may be, for example, 20 to 60 minutes at $37^{o}$C. The order of the contacting is not restricted in any way, and either of the ligand (1) and the combination may first be allowed to contact the antibody. Of course, both materials may be allowed to contact at the same time.

Subsequently, the system thereby obtained is allowed to make contact with a biotinyl enzyme. The biotinyl enzyme has a biotinyl group in its active site, and is preferably selected from propionyl CoA carboxylase, acetyl CoA carboxylase, pyruvate carboxylase, methylmalonyl CoA carboxylase, methylmalonyl CoA transcarboxylase, and methylcrotonyl CoA carboxylase.

It is sufficient that the biotinyl enzyme be allowed to make contact with the combination which has been allowed to make contact with the antibody. Thus, the enzyme may be added to the test solution prior to the reactions of the ligand (1) and the aforesaid combination with the antibody, or it may also be added after the reactions. The pH of the reaction medium is suitably 4 to 8.5, and the reaction temperature is suitably from 20 to $45^{o}$C. The contacting time may be, for example, 10-60 minutes at $37^{o}$C.

After these contactings, the amount of the biotinyl enzyme which does not react with the biotinyl enzyme inhibitor of the aforesaid combination is measured. The measurement may be carried out by following any convenient method. For example, in the case of propionyl CoA carboxylase, ADP produced during the reaction is allowed to react in the presence of pyruvate kinase and lactate dehydrogenase, and the decrease of NADH is colorimetrically determined. In the case of methylmalonyl CoA transcarboxylase, oxalacetic acid produced is converted to malic acid in the presence of malate dehydrogenase, and the decrease of NADH is measured. Where propionyl CoA carboxylase is used, ATP produced by a reverse reaction is allowed to react in the presence of luciferase and luciferin, and bioluminescence is produced. When this bioluminescence is measured by using a photon counter, an ATP concentration lower than $10^{-14}$M can be determined. This method is suitable for determining an extremely small amount of ligand (1).

From the foregoing it can be seen that the method of the invention enables a ligand (1) to be determined to a high degree of sensitivity. For example, when using a biotinyl enzyme and avidin, a ligand (1) in an amount of $10^{-11}$ gram can be determined. Furthermore, the operation of the present method is simple and, for example, the separation of a bound and a free material is not necessary. Hence ligand (1) can easily and inexpensively be determined.

The following examples illustrate this invention:

## EXAMPLE 1

(i) Preparation of Avidin-Theophylline Combination

50 Mg of 3-propionylcarboxytheophylline were suspended in 5 ml of dimethyl sulphoxide, and 25 mg of N-hydroxysuccinimide were added. The suspension was cooled to $4^{\circ}$C, and 50 mg of dicyclohexylcarbodiimide were added with stirring. The mixture produced was stirred for 2 hours, and allowed to stand overnight at $4^{\circ}$C. Precipitates were filtered off, and solvent was evaporated under reduced pressure. A small amount of ether was added to the residue, and crystalline materials which formed were collected on a glass filter. The crystalline materials were washed with ether, and 30 mg of theophylline succinimide ester was thereby obtained.

0132292

-8-

20 Mg of avidin were then dissolved in 4 ml of 0.1 M carbonate buffer solution of pH 8.0, and 200 μl of a 2.0 mg/mol solution of the above theophylline succinimide ester in dimethyl sulphoxide was added to this. The mixed solution was stirred for 2 hours at ambient temperature. The reaction mixture thus obtained was passed through a Sephadex G-25 column which had been previously equilibrated with 20 mM phosphate buffered saline solution of pH 7.0, and the absorbance of the effluent at 280 nm was measured. The first peak fractions were collected, and lyophilised to yield the required combination of theophylline and avidin.

(ii) Measurement of Theophylline

5 μl portions of theophylline solution showing theophylline concentrations of 0 μg/ml, 5 μg/ml, 10 μg/ml, 20 μg/ml, 40 μg/ml and 80 μg/ml were respectively mixed with 20 μl of the above combinations of theophylline-avidin solution and 25 μl of anti-theophylline rabbit IgG solution. This mixture was allowed to stand for 30 minutes at ambient temperature. 100 μl of a propionyl Co carboxylase solution were added, and allowed to stand for 15 minutes at 37°C.

Subsequently, 1.2 ml of a substrate solution A (containing 4 mM $MgCl_2$, 2 mM reduced glutathione, 2 mM ATP, 0.1 M KCl, 50 mM potassium hydrogencarbonate, 1 mM phosphoenolpyruvic acid, 2500 U/l pyruvate kinase, 3500 U/l lactate dehydrogenase, 0.15 mM NADH, 2 mM propionyl CoA, pH 8.0) was added to each test mixture, and the absorbance at 350 nm of the reaction solution produced was measured.

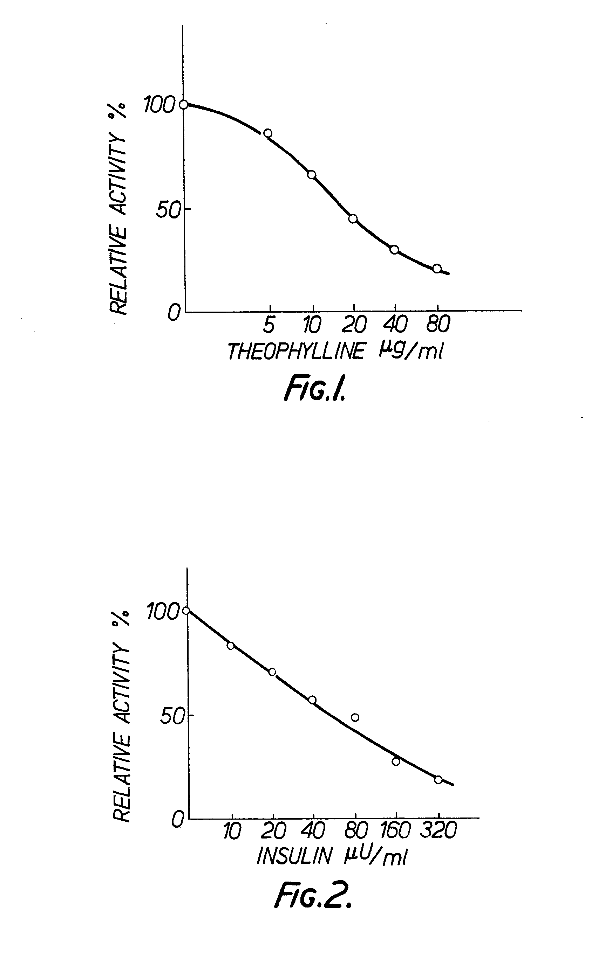

The relationship between the theophylline concentration and the activity of propionyl CoA carboxylase thus obtained is shown in Figure 1 of the accompanying drawings.

Next, immobilised avidin glass beads were prepared. For this purpose, glass beads with induced amino groups thereon were immersed in 2% glutaraldehyde

solution at $4^{\circ}C$ for 2 hours, and washed with 0.1 $Na_2CO_3$ solution of pH 9.0. Subsequently, the beads were immersed overnight in 0.1 M $Na_2CO_3$ solution of pH 9.0 containing 0.1% avidin at $4^{\circ}C$ and washed with 0.02 M phosphate buffered saline solution containing 1% BSA (bovine serum albumin). The washed beads were immersed in the same saline solution to obtain the required immobilised avidin glass beads.

The immobilised avidin glass beads were suspended in various human sera, separated off after 20 minutes, and 5 µl of each supernatant were collected. Using this supernatant, measurement was carried out in the same manner as above, and the theophylline concentration of each of the sera was determined by using the curve of Figure 1 as a calibration curve. For comparison purposes, the theophylline concentrations of the same sera were measured by the conventional radioimmunoassay (RIA) method. The results obtained are shown in the following Table 1.

## T a b l e   1

### Theophylline Concentration

| Serum | The method of the invention | RIA method |
|-------|------------------------------|------------|
| A1 | 5.1 mg/ml | 4.9 mg/ml |
| A2 | 11.6 " | 10.8 " |
| A3 | 17.6 " | 18.2 " |
| A4 | 3.1 " | 4.0 " |
| A5 | 7.8 " | 7.9 " |

## EXAMPLE   2

(i)   Preparation of SH-induced Streptoavidin

5 Mg of streptoavidin were dissolved in 1 ml of 0.1 M phosphate buffered 5 mM EDTA solution of pH 7.5. 100 µl of 9.6 mg/ml S-acetylmercaptosuccinic anhydride dioxane solution were added to the above solution, and allowed to react at 37$^{O}$C for one hour.   Subsequently, 110 µl of 1 M hydroxylamine solution pH 7.5 were added, and the reactants present were allowed to react at 37$^{O}$C for 30 minutes.   This reaction solution was introduced into a Sephadex G-25 column which had previously been equilibrated with 0.1 M phosphate buffered 1 mM EDTA solution pH 6.3, and gel filtration was carried out. The void fractions were collected, and the target SH-induced streptoavidin was obtained.

(ii)  Preparation of Insulin-Streptoavidin Combination

5 Mg of insulin were dissolved in 1 ml of 0.1 M phosphate buffer solution of pH 6.3.     0.2 Mg of 4-(maleimidomethyl    cyclohexane-1-carboxylic    acid) succinimide ester (CHM) were dissolved in 100  µl of dioxane, and added to the above insulin solution.   The mixture obtained was stirred at 37$^{O}$C for one hour, and then gel filtration was carried out by using a Sephadex G-25 column to remove unreacted CHMS.   Since CHM-induced insulin was passed through the column, the void fractions were collected, and the above SH-induced streptoavidin was added.   The mixture obtained was concentrated to 1 ml, and warmed at 30$^{O}$C for 1 hour.   Then, gel filtration was carried out by using Sephadex G-200, and the target combination was obtained.

(iii)  Measurement of Insulin

20   µl  portions  of  insulin  solutions  whose concentrations were 0  µU/ml, 10  µU/ml, 20  µU/ml, 40 µU/ml, 80 µU/ml, 160  µU/ml and 320 µU/ml were each placed in a test tube, and 50 µl of a solution containing 2.0 µg of the combination of insulin-streptoavidin were added to the contents of each test tube.   Subsequently, 50 µl of anti-insulin guinea pig IgG solution were added, and the contents of the test tubes were warmed at 37$^{O}$C for 30 minutes.    50  µl  of  a  solution  containing  40  µg  of methylmalonyl CoA transcarboxylase were then added, and the test tube contents were again warmed at 37$^{O}$C for 10 minutes.   1.0 ml of 50 mM tris-HCl buffer solution of pH 7.2 containing 5 mM pyruvic acid, 2000 U/l malate dehydrogenase, 0.25 mM NADH and 3 mM methylmalonyl CoA were added to each test tube, and the absorbance at 340 nm of the reaction solution was measured.

The relationship between the insulin concentration and the activity of methylmalonyl CoA transcarboxylase thus obtained is shown in Figure 2 of the accompanying drawings.

Next, immobilised avidin glass beads produced as described in Example 1, were added to various human sera, and kept therein at 37°C for 5 minutes. The supernatant present was then separated off by centrifugation, and supernatants were subjected to determination of their insulin concentration in the same manner as above. The insulin concentration of each serum was determined by using the curve of Figure 2 as the calibration curve, and the results obtained are shown in the following Table 2. Results obtained for the sera by the conventional RIA method are also shown in the same table.

### T a b l e   2
#### Insulin Concentration

| Serum | The method of the invention | | RIA method | |
|-------|------------|------|------|------|
| B1 | 9.5 | U/ml | 9.0 | U/ml |
| B2 | 27 | " | 26 | " |
| B3 | 110 | " | 107 | " |
| B4 | 26 | " | 22 | " |
| B5 | 241 | " | 230 | " |

### EXAMPLE 3

(i) Preparation of Combination of Avidin-Digoxin

5 Mg of digoxin were suspended in 0.3 ml of ethanol, and 0.3 of 0.1 M sodium metaperiodate was added to this. The mixture obtained was stirred at ambient temperature for 25 minutes. 6 µl of 1 M ethylene glycol were added to the mixture, and after 5 minutes, 5 mg of avidin dissolved in 300 μl of 0.1 M carbonate buffer solution of pH 9.5 were added to this. The pH of the solution was maintained at 9.0, and after 45 minutes, 3 mg of $NaBH_4$ were added and stirring for 3 hours was carried out. The pH was adjusted to 6.5 by adding 1 M formic acid and further stirring for 1 hour was effected. The solution was adjusted to pH 8.5 by adding 1 M $NH_4OH$, and dialysed against 20 mM phosphate buffered saline solution to yield 5 mg of the target combination of avidin-digoxin.

(ii) Measurement of Digoxin

10 μl samples of digoxin solutions whose concentrations were 0 ng/ml, 0.1 ng/ml, 0.5 ng/ml, 1 ng/ml, 5 ng/ml and 10 ng/ml respectively were each mixed with 20 μl of a solution containing 5 μg of the above combination of avidin-digoxin and 20 1 of a solution containing 10 μg of anti-digoxin rabbit IgG. 3 μg of propionyl CoA transcarboxylase were added to each mixture which was kept at $37^{O}C$ for 1 hour. 1.0 Ml of a substrate solution containing 2 mmol/l propionyl CoA, 30 mmol/l oxaloacetic acid, 0.2 mmol/l NADH, 2000 U/ml lactate dehydrogenase and 0.1 M tris-HCl, pH 7.5 was added to each reaction mixture, and the absorbance at 340 nm of the reaction solution was measured.

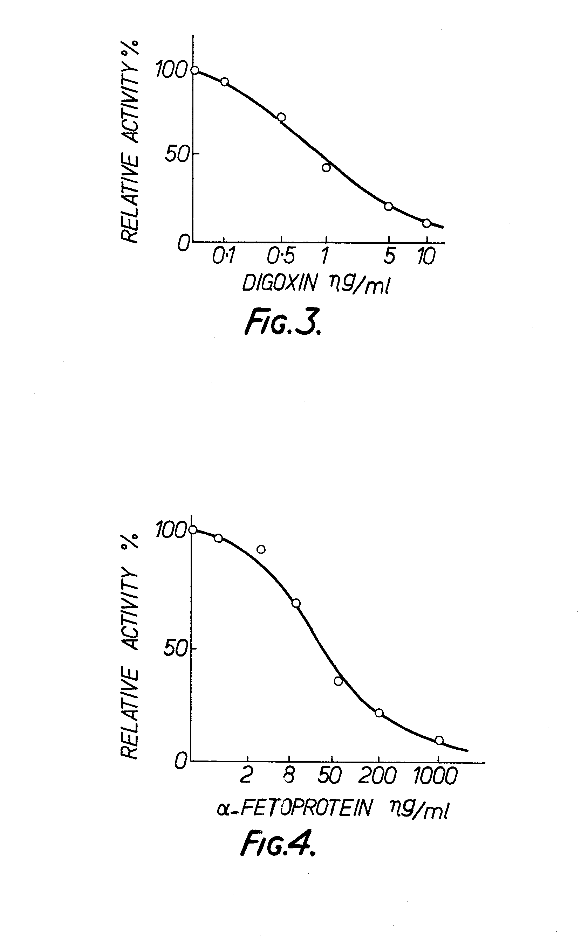

The relationship between the digoxin concentration and the activity of propionyl CoA transcarboxylase thus obtained is shown in Figure 3 of the accompanying drawings.

Avidin-bonded Sepharose 4B was then prepared. 5 G of CNBr-activated Sepharose (made by Pharmacia Fine Chemicals) were washed with 500 ml of 1 mM HCl, and further washed once with 0.2 M $NaHCO_3$-0.5 M NaCl solution of pH 8.0. The washed Sepharose was suspended in 0.1 M $NaHCO_3$-0.5 M NaCl solution of pH 8.0 containing 1% avidin, and stirred at ambient temperature for 2 hours. The target gel thus obtained was washed with 0.2 M tris HCl buffer solution of pH 8.5, and preserved in 0.1 M

-13-

0132292

tris HCl buffer solution of pH 8.0 containing 1% BSA.

Various human sera were passed through the column packed with the above avidin-bonded Sepharose 4B, and then the digoxin concentrations of the sera were measured in the same manner as above. The digoxin concentrations were determined by using the curve of Figure 3 as a calibration curve, and the results are shown in the following Table 3. The results obtained by the conventional RIA method are also shown in the same table.

## T a b l e 3

### Digoxin Concentration

| Serum | The method of the invention | RIA Method |
|-------|-----------------------------|------------|
| C1 | 0.35 ng/ml | 0.32 ng/ml |
| C2 | 0.81 " | 0.90 " |
| C3 | 1.20 " | 1.11 " |
| C4 | 2.24 " | 2.36 " |
| C5 | 0.15 " | 0.13 " |

### EXAMPLE 4

(i) Preparation of CHM-induced α-Fetoprotein

5 MG of α-fetoprotein were dissolved in 1 ml of 0.1 M phosphate buffer solution of pH 6.3. 100 μl of 2 mg/ml CHM solution in dimethyl formamide solution were added to this solution, and allowed to react at $30^{\circ}C$ for 1 hour. The reaction mixture was then passed through a Sephadex G-25 column which had previously been equilibrated with 0.1 M phosphate buffer solution pH 6.3 containing 1 mM EDTA, and void fractions containing CHM-induced α-fetoprotein were collected.

(ii) Preparation of SH-induced Streptoavidin

5 Mg of streptoavidin were dissolved in 0.1 M phosphate buffer solution of pH 7.5 containing 5 mM EDTA. 100 μl of a solution of S-acetylmercaptosuccinic anhydride in dimethyl formamide in a concentration of 9 mg/ml were added to this solution, and the mixed solution was warmed at 37°C for 1 hour. Subsequently, 110 µl of 1 M $NH_2OH$ pH 7.5 were added, and the reaction mixture was kept at 37°C for a further 30 minutes. This reaction mixture was introduced into a Sephadex G-25 column, and void fractions were collected to yield SH-induced streptoavidin.

(iii) Preparation of α-Fetoprotein-Streptoavidin Combination

The void fractions containing CHM-induced α-fetoprotein obtained in step (i) were mixed with the void fractions containing SH-induced streptoavidin obtained in step (ii). The mixture obtained was concentrated to 1 ml, and warmed at 37°C for 1 hour. The reaction mixture was then introduced into a Sephadex G-150 column, and gel filtration was carried out. The void fractions were collected, and the target combination was obtained.

(iv) Measurement of α-Fetoprotein

20 µl samples of α-fetoprotein solution whose concentrations were 0 ng/ml, 0.5 ng/ml, 2 ng/ml, 8 ng/ml, 50 ng/ml, 200 ng/ml and 1000 ng/ml were each placed in a test tube, and 20 µl of a solution containing 1000 ng/ml of the combination of α-fetoprotein-streptoavidin prepared in step (III) and 2 µg/ml anti-α-fetoprotein goat antibody were added to each solution. 50 µl of 10 µg/ml pyruvate carboxylase was further added, and the contents of the test-tubes were warmed at 37°C for 1 hour. Subsequently, 0.1 M tris-HCl buffer solution of pH 7.8 containing 4 mM $MgCl_2$, 5 mM ADP, 10 mM oxaloacetic acid, 10 mM pyrophosphoric acid and 0.1 M KCl was added to each solution as a substrate solution and reaction at 37°C was allowed to take place for 20 minutes. The reaction was stopped by adding 100 µl of 10 µg/ml avidin solution. 100 µl of luciferin-luciferase solution were added to 400 µl of this solution, and bioluminescence at 565 nm was measured.

The results obtained are shown in Figure 4 of the accompanying drawings.  The graph produced was suitable for use as a calibration curve in the determination of $\alpha$-fetoprotein concentrations of sera.

Claims:

1. A method of measuring a biological ligand, characterised by allowing there to coexist in an aqueous medium a system comprising (A) a ligand (1) to be measured, (B) a combination of a biotinyl enzyme inhibitor and a ligand (2) having an antigenic determinant which is also an antigenic determinant of ligand (1) and (C) an antibody capable of reacting with said antigenic determinant, contacting said system with a biotinyl enzyme, measuring the residual biotinyl enzyme activity and utilising the measurement thereby obtained as a measure of the biological ligand.

2. A method as claimed in claim 1, wherein said ligand (1) and said ligand (2) are selected from digoxin, theophylline, phenytoin, penicillin, amikacin, gentamicin, insulin, TSH, T4, prostaglandin, IgG, α-fetoprotein, glycolipids, HBs antigen and carcinoembryonic antigens.

3. A method as claimed in claim 1 or 2, wherein said ligand (1) and said ligand (2) are the same substance.

4. A method as claimed in any preceding claim, wherein said biotinyl enzyme inhibitor is avidin, streptoavidin or a derivative thereof capable of binding to biotin.

5. A method as claimed in any one of the preceding claims, wherein said antibody is a fragment of immunoglobulin.

6. A method as claimed in any one of claims 1 to 4, wherein said antibody is a monoclonal antibody.

7. A method as claimed in any one of the preceding claims, wherein said biotinyl enzyme is selected from propionyl CoA carboxylase, acetyl CoA carboxylase, pyruvate carboxylase, methylmalonyl CoA carboxylase, methylmalonyl CoA transcarboxylase and methylcrotonyl CoA carboxylase.

8. A method as claimed in any one of the preceding claims wherein, in measuring the biotinyl enzyme activity, a reaction is carried out which produces photoluminescence which is measured quantitatively using a photon counter.

9. A method as claimed in any one of the preceding claims, wherein from 1 to 5 moles of said antibody are present for each 1 to 5 moles of said combination.

10. A method as claimed in any one of the preceding claims, wherein said aqueous solution has a pH of from 4 to 8.5 and a temperature of from 20 to 45$^{o}$C.

11. A method as claimed in any one of the preceding claims, wherein the enzyme is added to the aqueous medium prior to the reaction of said combination with the antibody.

1/2

0132292

Fig.1.

Fig.2.

0132292

FIG.3.

FIG.4.